(19) Europäisches Patentamt / European Patent Office / Office européen des brevets

(11) **EP 4 733 311 A1**

(12) **EUROPEAN PATENT APPLICATION**

(43) Date of publication:
29.04.2026 Bulletin 2026/18

(21) Application number: 25210991.3

(22) Date of filing: 24.10.2025

(51) International Patent Classification (IPC):
*C07K 7/08* (2006.01)        *C40B 40/10* (2006.01)

(52) Cooperative Patent Classification (CPC):
**C07K 7/08; C40B 40/10;** G01N 33/582;
G01N 33/6896; G01N 2800/285

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 24.10.2024 US 202463711452 P

(71) Applicant: **HiPep Laboratories**
**Kyoto 602-8158 (JP)**

(72) Inventors:
• **NOKIHARA, Kiyoshi**
**Kyoto 602-8158 (JP)**
• **TOMINAGA, Yuki**
**Kyoto 602-8158 (JP)**

(74) Representative: **Mewburn Ellis LLP**
**Aurora Building**
**Counterslip**
**Bristol BS1 6BX (GB)**

(54) **STRUCTURE OF FLUORESCENTLY LABELED PEPTIDES USEFUL FOR DIFFERENTIATING MULTIPLE SCLEROSIS**

(57)    Disclosed is a fluorescently labeled peptide library useful for diagnosing multiple sclerosis. The fluorescently labeled peptide library comprising fluorescently labeled peptides represented by the following formula:

β-**Loop**        αTAMRA- KKITV-$X_1X_2X_3X_4$- KTYTE- GC- $NH_2$

wherein $X_1$, $X_2$, $X_3$ and $X_4$ are independently arbitrary amino acid residues, and the peptides are immobilized on a chip.

FIG. 1

## Description

Technical Field

The present invention relates to the structure of fluorescently labeled peptides used for differentiating multiple sclerosis using PepTenChip® system.

Background Art

In methods for diagnosing lesion states using body fluids as samples, a peptide microarray (referred to as PepTenChip®) is used, where numerous fluorescently labeled structured peptides are arrayed and immobilized on an amorphous carbon substrate. The inventors have discovered that the secondary structure of the immobilized peptides is crucial for recognizing the assay samples (analytes). They have synthesized and hold a library of numerous fluorescently labeled peptides that form three types of structures as shown in the following Formula 1.

**α-Helix**  αTAMRA-G-$X_1X_2X_3X_4X_5X_6X_7X_8X_9X_{10}X_{11}X_{12}X_{13}X_{14}$-GC-NH$_2$  **β-Sheet**  CG-$X_1X_2X_3X_4X_5X_6X_7X_8$-K(αTAMRA)-G-NH$_2$ **β-Loop** αTAMRA-KKITV-$X_1X_2X_3X_4$-KTYTE-GC-NH$_2$  Formula 1.

In the formula above, the portion represented by X consists of independent, arbitrary amino acid residues. Each structural peptide is immobilized on the amorphous carbon substrate via the sulfhydryl group of a Cys residue attached at either the C-terminus or N-terminus. Gly residues inserted before and after the Cys residue serve as spacers, and the part indicated by TAMRA, 5,6-Carboxytetramethylrhodamine as a fluorescent label.

Summary of the Invention

## Problems to be Solved by the Invention

Multiple sclerosis (MS) is a central nervous system demyelinating disease characterized by lesions in the brain, spinal cord, and optic nerves. It is marked by recurrent episodes of remission and relapse and remains an incurable, nationally designated intractable disease with unknown causes. Globally, approximately 2.3 million people are affected by MS, with about 21,000 cases reported in Japan in 2020 (including neuromyelitis optica [NMO]), and the number is increasing year by year (Source: Ministry of Health, Labour and Welfare, Health Administration Report). There is no established method for diagnosing this disease; generally, MRI, cerebrospinal fluid tests, and antibody tests for disease classification are performed, with diagnosis further applying the McDonald criteria. This criterion is used after excluding other possibilities through cerebrospinal fluid and antibody tests. It is also known that among MS cases diagnosed by the McDonald criteria, there are typical forms and atypical forms (Atypical MS) that exhibit different clinical pictures. Treatment responsiveness for Atypical MS differs from that of typical MS, making careful differentiation crucial. Additionally, since there are no specific biomarkers for MS, reliance on clinical features and MRI for diagnosis has sometimes led to misdiagnoses, highlighting the need for the development of objective diagnostic methods.

## Means for Solving the Problem

To address the aforementioned issues, the inventors have developed a new, safer, and more convenient testing method using the PepTenChip® system, a novel microarray technology for analyzing cerebrospinal fluid. Typical diagnostic methods using body fluids as analytes is one-to-one correspondence, between disease responsible materials (markers) and detection agents such as application of antigen-antibody reactions. This approach is effective when marker molecules are well-defined, but it is not applicable when the marker molecules are unknown. The inventors focused on the presence of various proteins in body fluids and developed a technique called peptide fingerprinting, which involves designing structure-recognizing peptides based on the amino acid sequences of protein recognition regions. These fluorescently labeled structure-recognizing peptides (capturing molecules) are arranged in an array, and changes in fluorescence intensity before and after contact with samples and the difference upon sample binding can be used for detection. This method is similar to antibody chips but is distinguished by its non-1:1 detection and patterning-based detection of samples, allowing for detection regardless of the sample type. The invention applies this technology to cerebrospinal fluid, providing peptide structures that aid in differentiating multiple sclerosis.

## Effects of the Invention

The invention allows for the differentiation of multiple sclerosis pathology using only a small amount of cerebrospinal fluid. It also demonstrates the importance of peptide structure and type in the microarray for classification, suggesting that by adjusting the types of peptides used, the microarray could be applied to various diseases.

Brief Description of the Drawings

Figure 1 shows the structure of the β-Loop peptides used in the examples below. Dotted line indicates ionic interactions.

Figure 2 shows the results of cerebrospinal fluid classification using the identified peptide group.

**Mode for Carrying out the Invention**

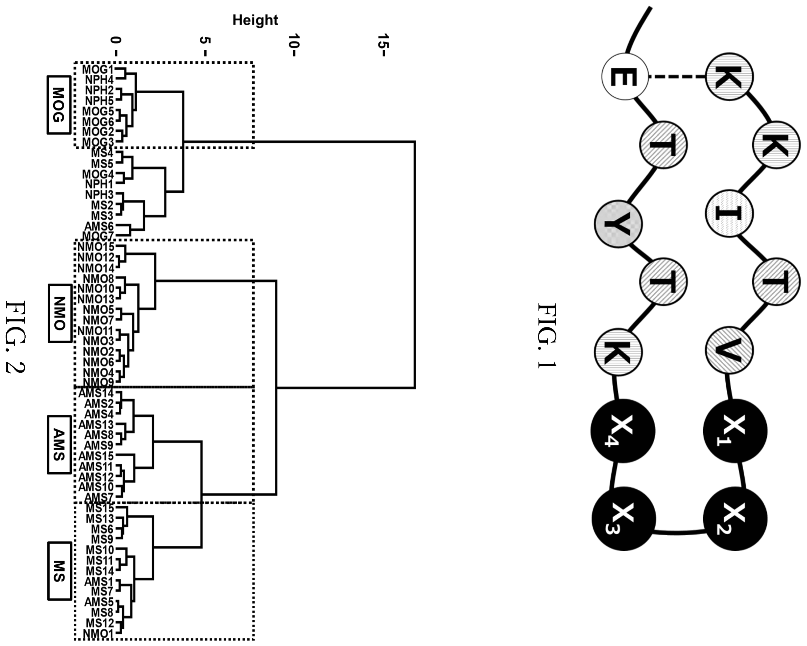

The structural peptides used to manufacture the peptide microarray in this invention are those forming a β-Loop structure as represented by the formula. The β-Loop structure forms a loop, with four amino acid residues present in the loop portion, as shown in the figures. The inventors identified that differences in the side-chain structures of the four amino acid residues in the loop are crucial for applying PepTenChip to cerebrospinal fluid diagnostics. They created a peptide microarray with a β-Loop library of 184 types, assayed it with cerebrospinal fluid samples with known pathology, and performed classification based on changes in fluorescence intensity. It was found that structures containing multiple aromatic amino acids in the loop are important for classification (Figure 2). The analysis indicated that a structure with at least three aromatic amino acids in the loop is desirable, and the presence of Tyr as an aromatic amino acid is crucial for differentiating cerebrospinal fluid.

Table 1 Sequence analysis results of peptide probes identified in the examples

| Probe# as capturing molecules | Sequence ($X_1X_2X_3X_4$) | $X_1$ | $X_2$ | $X_3$ | $X_4$ |
|---|---|---|---|---|---|
| 250 | EYHW | Glu | **Tyr** | **His** | **Trp** |
| 324 | PFHY | Pro | **Phe** | **His** | **Tyr** |
| 349 | WFYL | **Trp** | **Phe** | **Tyr** | Leu |
| 365 | YWQF | **Tyr** | **Trp** | Gln | **Phe** |

X denotes amino acid residues forming the loop structure shown in Figure 1.
Aromatic amino acids are indicated in bold.

In other words:

1. It is preferable that the loop structure of β-Loop peptides contains three or more aromatic amino acids.
2. It is preferable that Tyr is included as an aromatic amino acid forming the loop structure.

**Example 1: Synthesis and Preparation of Peptide Microarrays**

The major reagents used for peptide synthesis included amino acid derivatives, 2-(1H-Benzotriazole-1-yl)-1,1,3,3-tetramethyluronium hexafluorophosphate (HBTU), 1-Hydroxybenzotriazole (HOBt), and fluorescent dyes such as 5(6)-Carboxyfluorescein (FAM) and 5(6)-Carboxytetramethylrhodamine (TAMRA), as well as ε-maleimidocaproic acid (EMCA). These were obtained from HiPep Laboratories (Kyoto, Japan). The solid support used was TentaGel® S-RAM (Rapp Polymere GmbH, Tübingen, Germany). Other reagents and solvents were purchased from Nakalai Tesque, Inc. (Kyoto, Japan). Water was prepared using a Milli-Q® system (Merck-Millipore, Tokyo, Japan). Peptide quality evaluation was performed using an online LC-MS system (LC: Agilent 1100, Agilent Technologies Inc., MS: HCTultra, Bruker Japan K.K., Yokohama). For preparing peptide solutions for microarray construction, 384-well microtiter plates (Thermo Fisher Scientific K.K., Yokohama, Japan) were used. Peptides were synthesized using Fmoc solid-phase synthesis with an automated synthesizer PSSM-8 (Shimadzu Corporation, Kyoto, Japan) and manual synthesizer, PetiSyzer® (HiPep Laboratories), and were cleaved from the resin. After purification with a column (50 i.d. × 250 mm), 1 mg of synthesized fluorescently labeled peptide was dissolved in 180 μL of 1,1,1,3,3,3-hexafluoro-2-propanol (HFI-P)/$H_2$O/AcOH = 1/1/1 (v/v/v) and then diluted with Dimethyl sulfoxide (DMSO) to a total volume of 400 μL to prepare the stock solution. The concentration of the stock solution was determined by UV measurement using NanoDrop™ (Thermo Fisher Scientific). The stock solution was diluted 200-fold with methanol, and absorbance at 450-600 nm was measured at 25°C. The concentration of the fluorescently labeled peptide in the stock solution was calculated from the absorbance at the maximum wavelength of TAMRA (545 nm) and its absorption coefficient ($\varepsilon$ = 91,000). The prepared stock solution was stored at -80°C until used for arraying.

Microarrays were prepared by a NanoPrint™ LM-60 microarrayer (Arrayit Corp., CA, USA) with Stealth Pin 946MP4 (spot diameter 160 $\mu$m, Arrayit Corp.). Fluorescently labeled peptides were immobilized by dilution of 1 mM peptide solutions 10-fold with 1% acetic acid. The peptide solution was dispensed into a 384-well microtiter plate (Optiplate™ 384-F, Perkinelmer Japan, Yokohama, Japan) and set into the NanoPrint™ LM-60 for arraying. The array was created using an amorphous carbon substrate with three derivatized cover glass-sized regions. After arraying, the substrate was washed three times each with 2-propanol/$H_2O$ = 1/1 solution and ultrapure water, then spin drying. The resulting substrate was examined using a fluorescence detection device (PepTenCam, HiPep Laboratories) to ensure that the spots were in good condition before use in assays.

By the method described above, 184 peptides of the general formula

$\beta$-**Loop** $\qquad$ $\alpha$TAMRA-KKITV-$X_1 X_2 X_3 X_4$-KTYTE-GC-$NH_2$

were synthesized and immobilized on the amorphous carbon substrates. The four residues represented by $X_1$, $X_2$, $X_3$ and $X_4$ in the general formula of the synthesized 184 peptides are shown in Table 2 below.

**Table 2-1**

| SN | Peptide# | $X_1$ | $X_2$ | $X_3$ | $X_4$ |
|---|---|---|---|---|---|
| 1 | L0002 | H | D | F | E |
| 2 | L0008 | W | F | D | E |
| 3 | L0009 | D | Y | F | E |
| 4 | L0023 | H | D | W | E |
| 5 | L0045 | E | Y | D | W |
| 6 | L0046 | F | G | H | D |
| 7 | L0052 | D | W | G | F |
| 8 | L0053 | G | Y | D | F |
| 9 | L0054 | L | D | H | F |
| 10 | L0055 | F | H | P | D |
| 11 | L0056 | H | F | D | Q |
| 12 | L0057 | H | D | F | S |
| 13 | L0058 | D | R | F | H |
| 14 | L0059 | F | H | D | W |
| 15 | L0060 | F | D | H | Y |
| 16 | L0065 | W | F | D | L |
| 17 | L0066 | Y | D | L | F |
| 18 | L0070 | W | P | D | F |
| 19 | L0071 | P | F | Y | D |
| 20 | L0074 | Q | D | F | W |
| 21 | L0075 | Y | F | D | Q |
| 22 | L0077 | D | R | F | W |
| 23 | L0078 | F | D | Y | R |
| 24 | L0079 | F | D | S | W |
| 25 | L0080 | S | D | Y | F |
| 26 | L0081 | Y | W | D | F |
| 27 | L0087 | G | W | D | H |

(continued)

| SN | Peptide# | $X_1$ | $X_2$ | $X_3$ | $X_4$ |
|----|----------|-------|-------|-------|-------|
| 28 | L0109 | Y | D | W | G |
| 29 | L0114 | D | L | H | W |
| 30 | L0119 | W | H | D | P |
| 31 | L0120 | Y | P | D | H |
| 32 | L0123 | W | H | D | Q |

Table 2-2

| SN | Peptide# | $X_1$ | $X_2$ | $X_3$ | $X_4$ |
|----|----------|-------|-------|-------|-------|
| 33 | L0126 | D | R | W | H |
| 34 | L0127 | R | H | Y | D |
| 35 | L0128 | D | W | H | S |
| 36 | L0130 | H | D | Y | W |
| 37 | L0145 | D | W | Y | L |
| 38 | L0155 | D | P | Y | W |
| 39 | L0161 | D | W | Q | Y |
| 40 | L0164 | Y | R | D | W |
| 41 | L0165 | D | W | S | Y |
| 42 | L0166 | F | E | G | H |
| 43 | L0172 | G | W | E | F |
| 44 | L0173 | E | G | F | Y |
| 45 | L0174 | H | F | E | L |
| 46 | L0175 | P | H | F | E |
| 47 | L0176 | E | Q | H | F |
| 48 | L0177 | H | R | E | F |
| 49 | L0178 | E | F | H | S |
| 50 | L0179 | H | E | W | F |
| 51 | L0180 | E | H | F | Y |
| 52 | L0185 | F | L | E | W |
| 53 | L0186 | Y | E | L | F |
| 54 | L0190 | P | W | E | F |
| 55 | L0191 | E | P | F | Y |
| 56 | L0194 | Q | E | F | W |
| 57 | L0195 | E | F | Y | Q |
| 58 | L0197 | W | E | F | R |
| 59 | L0198 | R | F | E | Y |
| 60 | L0199 | E | S | W | F |
| 61 | L0200 | Y | S | F | E |
| 62 | L0201 | W | F | Y | E |
| 63 | L0207 | G | E | W | H |

(continued)

| SN | Peptide# | X₁ | X₂ | X₃ | X₄ |
|---|---|---|---|---|---|
| 64 | L0229 | W | E | Y | G |
| 65 | L0234 | L | E | H | W |

Table 2-3

| SN | Peptide# | X₁ | X₂ | X₃ | X₄ |
|---|---|---|---|---|---|
| 66 | L0239 | E | P | H | W |
| 67 | L0240 | H | P | E | Y |
| 68 | L0243 | W | Q | E | H |
| 69 | L0246 | E | H | R | W |
| 70 | L0247 | R | H | E | Y |
| 71 | L0248 | W | H | S | E |
| 72 | L0250 | E | Y | H | W |
| 73 | L0265 | W | Y | L | E |
| 74 | L0275 | Y | W | P | E |
| 75 | L0281 | Q | W | E | Y |
| 76 | L0284 | W | E | R | Y |
| 77 | L0285 | E | Y | S | W |
| 78 | L0286 | G | F | L | H |
| 79 | L0287 | F | G | H | P |
| 80 | L0288 | G | F | H | Q |
| 81 | L0289 | R | H | F | G |
| 82 | L0290 | H | F | S | G |
| 83 | L0291 | W | H | G | F |
| 84 | L0292 | F | H | Y | G |
| 85 | L0297 | G | W | L | F |
| 86 | L0298 | L | Y | G | F |
| 87 | L0302 | P | G | W | F |
| 88 | L0303 | F | Y | P | G |
| 89 | L0306 | W | Q | G | F |
| 90 | L0307 | Y | G | F | Q |
| 91 | L0309 | G | W | R | F |
| 92 | L0310 | R | F | G | Y |
| 93 | L0311 | S | W | F | G |
| 94 | L0312 | F | Y | G | S |
| 95 | L0313 | G | Y | W | F |
| 96 | L0314 | H | F | P | L |
| 97 | L0315 | Q | H | F | L |
| 98 | L0316 | F | H | L | R |

6

Table 2-4

| SN | Peptide# | X₁ | X₂ | X₃ | X₄ |
|---|---|---|---|---|---|
| 99 | L0317 | L | H | F | S |
| 100 | L0318 | W | L | H | F |
| 101 | L0319 | Y | F | L | H |
| 102 | L0320 | H | Q | F | P |
| 103 | L0321 | P | H | R | F |
| 104 | L0322 | F | P | H | S |
| 105 | L0323 | P | H | W | F |
| 106 | L0324 | P | F | H | Y |
| 107 | L0325 | H | R | F | Q |
| 108 | L0326 | S | Q | F | H |
| 109 | L0327 | W | F | Q | H |
| 110 | L0328 | Y | Q | F | H |
| 111 | L0329 | H | R | S | F |
| 112 | L0330 | W | H | R | F |
| 113 | L0331 | F | Y | H | R |
| 114 | L0332 | F | S | W | H |
| 115 | L0333 | S | F | H | Y |
| 116 | L0334 | Y | H | F | W |
| 117 | L0338 | F | L | P | W |
| 118 | L0339 | P | L | Y | F |
| 119 | L0342 | F | Q | W | L |
| 120 | L0343 | Q | F | L | Y |
| 121 | L0345 | R | W | F | L |
| 122 | L0346 | L | R | F | Y |
| 123 | L0347 | F | L | S | W |
| 124 | L0348 | Y | S | L | F |
| 125 | L0349 | W | F | Y | L |
| 126 | L0352 | W | P | Q | F |
| 127 | L0353 | Q | F | Y | P |
| 128 | L0355 | P | W | F | R |
| 129 | L0356 | Y | F | P | R |
| 130 | L0357 | S | P | W | F |

Table 2-5

| SN | Peptide# | X₁ | X₂ | X₃ | X₄ |
|---|---|---|---|---|---|
| 131 | L0358 | Y | S | F | P |
| 132 | L0359 | F | W | P | Y |
| 133 | L0361 | W | F | Q | R |
| 134 | L0362 | Q | F | Y | R |

(continued)

| SN | Peptide# | $X_1$ | $X_2$ | $X_3$ | $X_4$ |
|---|---|---|---|---|---|
| 135 | L0363 | F | Q | W | S |
| 136 | L0364 | F | Y | S | Q |
| 137 | L0365 | Y | W | Q | F |
| 138 | L0366 | R | F | W | S |
| 139 | L0367 | Y | S | F | R |
| 140 | L0368 | R | Y | W | F |
| 141 | L0369 | W | F | S | Y |
| 142 | L0374 | G | W | L | H |
| 143 | L0379 | W | H | P | G |
| 144 | L0380 | Y | H | P | G |
| 145 | L0383 | W | Q | H | G |
| 146 | L0386 | R | H | W | G |
| 147 | L0387 | Y | H | R | G |
| 148 | L0388 | W | S | H | G |
| 149 | L0390 | Y | H | G | W |
| 150 | L0405 | Y | G | L | W |
| 151 | L0415 | Y | W | G | P |
| 152 | L0421 | Y | W | G | Q |
| 153 | L0424 | G | W | Y | R |
| 154 | L0425 | S | Y | W | G |
| 155 | L0429 | L | W | P | H |
| 156 | L0430 | Y | L | H | P |
| 157 | L0433 | Q | L | H | W |
| 158 | L0436 | W | R | L | H |
| 159 | L0437 | Y | H | L | R |
| 160 | L0438 | L | W | S | H |
| 161 | L0440 | W | L | H | Y |
| 162 | L0443 | P | H | Q | W |
| 163 | L0446 | W | H | P | R |

Table 2-6

| SN | Peptide# | $X_1$ | $X_2$ | $X_3$ | $X_4$ |
|---|---|---|---|---|---|
| 164 | L0447 | R | P | H | Y |
| 165 | L0448 | P | S | H | W |
| 166 | L0449 | S | P | H | Y |
| 167 | L0450 | H | P | Y | W |
| 168 | L0452 | Q | H | R | W |
| 169 | L0453 | Y | R | Q | H |
| 170 | L0454 | W | H | Q | S |

(continued)

| SN | Peptide# | $X_1$ | $X_2$ | $X_3$ | $X_4$ |
|---|---|---|---|---|---|
| 171 | L0456 | H | W | Q | Y |
| 172 | L0457 | H | W | S | R |
| 173 | L0459 | Y | R | H | W |
| 174 | L0460 | H | W | Y | S |
| 175 | L0470 | P | W | L | Y |
| 176 | L0476 | Q | L | Y | W |
| 177 | L0479 | R | W | Y | L |
| 178 | L0480 | W | S | L | Y |
| 179 | L0486 | Q | Y | P | W |
| 180 | L0489 | P | Y | R | W |
| 181 | L0490 | S | W | Y | P |
| 182 | L0493 | Q | Y | W | R |
| 183 | L0494 | Y | S | Q | W |
| 184 | L0495 | R | S | W | Y |

**Example 2: Assay with Samples**

PBS (10 $\mu$L) was applied to each of the three blocks on the peptide microarray and covered with a cover glass to spread the solution over the entire array, then incubated at room temperature in the dark for 30 minutes. After incubation, fluorescence images were measured using a fluorescence detection device and quantified with the ArrayPro® analyzer as $I_0$. The substrate was then washed three times each with 2-propanol/$H_2O$ = 1/1 solution and ultrapure water, and spin drying. Subsequently, cerebrospinal fluid samples, as listed in the table, were treated similarly to PBS, and fluorescence images were measured and quantified as $I_1$. The fluorescence intensity change was calculated using the formula 2 from the measured values $I_0$ and $I_1$, and multivariate analysis was performed using the statistical software "R".

$$\text{Formula 2: } \Delta\frac{I_1}{I_0} = \frac{I_1 - I_0}{I_0}$$

**Example 3: Classification of Cerebrospinal Fluid Samples**

The fluorescence intensity change data obtained from cerebrospinal fluid samples in Example 2 were converted to CSV format and analyzed using the statistical software R. Due to the differences in samples, data standardization was performed prior to statistical analysis. To determine the appropriate number of clusters for classification, silhouette analysis was conducted, and cluster analysis was performed based on the resulting number of clusters. The results of the cluster analysis are shown in Figure 2.

Figure 2, X-axis is the sample obtained from patients. Figure shows classification of diseases into each cluster. Abbreviations: MS: Multiple Sclerosis; AMS: Atypical-MS; NMO: Neuromyelitis Optica; MOG: anti-myelin oligodendrocyte glycoprotein antibody-positive optic neuritis; NPH: Normal Pressure Hydrocephalus.

**Claims**

1.   A fluorescently labeled peptide library comprising fluorescently labeled peptides represented by the following formula:

$\beta$-**Loop**          $\alpha$TAMRA-KKITV-$X_1X_2X_3X_4$-KTYTE-GC-$NH_2$

wherein $X_1$, $X_2$, $X_3$ and $X_4$ are independently arbitrary amino acid residues, and
said peptides are immobilized on a chip.

2. The fluorescently labeled peptide library according to claim 1, wherein three or more of the four residues represented by $X_1$, $X_2$, $X_3$ and $X_4$ forming the loop structure involves aromatic amino acids.

3. The fluorescently labeled peptide library according to claim 2, wherein one of the four residues is Tyr.

4. The fluorescently labeled peptide library according to claim 3, wherein the four residues are selected from the group consisting of: EYHW, PFHY, WFYL and YWQF.

5. The fluorescently labeled peptide library according to any one of claims 1 to 4, wherein the total number of the fluorescently labeled peptides immobilized on the chip is from 20 to 184, preferably from 20 to 50.

6. The fluorescently labeled peptide library according to any one of claims 1 to 5, wherein among the four residues, two residues are aromatic amino acids, preferably three or more residues are composed of aromatic amino acids.

7. The fluorescently labeled peptide library according to claim 5, wherein at least 70% peptides immobilized on the chip comprises two or more aromatic amino acids as the four residues.

8. The fluorescently labeled peptide library according to claim 5, comprising immobilized polypeptides whose four residues are EYHW, PFHY, WFYL and YWQF, respectively.

9. The fluorescently labeled peptide library according to claim 5, wherein at least 90% peptides immobilized on the chip have the four residues selected from those shown in Table 2.

FIG. 1

FIG. 2

EP 4 733 311 A1

Europäisches
Patentamt
European
Patent Office
Office européen
des brevets

**EUROPEAN SEARCH REPORT**

Application Number

EP 25 21 0991

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TOMINAGA Y ET AL: "Recognition of a monoclonal antibody against a small molecular weight antigen by monitoring the antigen-antibody reaction using fluorescence labeled structured pept", BIOORGANIC & MEDICINAL CHEMISTRY LETTERS, vol. 25, no. 3, 9 December 2014 (2014-12-09), pages 611-615, XP029131270, DOI: 10.1016/J.BMCL.2014.12.009 | 1,5 | INV. C07K7/08 C40B40/10 |
| A | * the whole document, in particular abstract; figures 1, 2; table 3 * -& Tominaga Y ET AL: "Supplementary data for: Recognition of a monoclonal antibody against a small molecular weight antigen by monitoring the antigen-antibody reaction using fluorescence labeled structured peptides.", Bioorg. Med. Chem. Lett., 1 February 2015 (2015-02-01), XP093373750, Retrieved from the Internet: URL:https://ars.els-cdn.com/content/image/1-s2.0-S0960894X14013067-mmc1.doc * table S3 * | 2-4,6-9 | |
| X,P | TOMINAGA Y ET AL: "Development of a new biodetection system independent of known marker molecules using a novel material for microarrays made from amorphous carbon substrates", ANALYTICAL METHODS, vol. 17, no. 22, 9 May 2025 (2025-05-09), pages 4590-4598, XP093373784, DOI: 10.1039/D5AY00426H Retrieved from the Internet: URL:https://pubs.rsc.org/en/content/articlepdf/2025/ay/d5ay00426h> | 1 | TECHNICAL FIELDS SEARCHED (IPC) C07K C12N C40B G01N |
| A,P | * the whole document * | 2-9 | |

The present search report has been drawn up for all claims

| Place of search | Date of completion of the search | Examiner |
|---|---|---|
| Munich | 9 March 2026 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)